# EUROPEAN PATENT APPLICATION

(11) **EP 3 106 192 A1**
(43) Date of publication of application: **21.12.2016**
(21) Application number: 15172499.4
(22) Date of filing: 17.06.2015
(51) Int. Cl.: A61M 5/315

(54) **DOSE SETTING AND DOSE LIMITING MECHANISM FOR AN INJECTION DEVICE AND METHOD FOR PROGRAMMING THE DOSE LIMITATION**

(71) Applicant: TecPharma Licensing AG, 3401 Burgdorf (CH)
(72) Inventor: HOSTETTLER, Patrick, 3415 Hasle (CH); STREIT, Ursina, 3322 Schönbühl (CH); SCHENKER, Susanne, 4900 Langenthal (CH)

(57) **Abstract**

A dose setting mechanism for an injection device that limits the setting of a dose beyond a predetermined amount of medication. The mechanism is based on a first stop element (400) that reciprocates between a first position and a second position during the dose setting and dose delivery step, and a second stop element (300) that moves unidirectionally during dose delivery. The first and second stop elements both have limiters (301)(401)and the axial or angular distance between the two limiters is reduced during dose setting and remains contact during dose delivery whereby the setting of a dose exceeding a predetermined amount of medication is prevented when the two limiters are engaged. Furthermore, a method for assembly and programming the predetermined amount of medication that can be dispensed from the injection device is presented.

## Description

The invention applies to a dose setting mechanism for an injection device preventing the setting of a dose which exceeds an amount of product present in a cartridge. The dose setting mechanism and the injection device with the dose setting mechanism serves the purpose of dispensing a fluid product, particularly a medicament.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Injection devices such as injection pens are known from the state of the art. Individual doses of a product can be set and subsequently dispensed with such injection devices. This procedure can be repeated several times and each time a specific dose can be set and dispensed from the device using the dose setting mechanism. Because the amount of medicament present in the cartridge is limited, namely for example 300 International Units (IU) of insulin, it can happen that a dose is set which is above the amount that is remaining in, and can be dispensed from the cartridge. This could result in that for example 35 IU units are set by the user whereas with the subsequent injection, only 20 IU can be dispensed. The difference of 15IU is missing for the therapy and a user of the device might not notice this, which can lead to hazardous adverse dosing.

It is therefore advantageous for the user to have a dosing mechanism including a dose limiting mechanism, which limits the accumulated doses set and dispensed from the device equal to an amount of medication present in the cartridge. The final dose set before emptying the cartridge is limited to the actual amount of medication still present in the cartridge and the user who, for example, intended to set a dose of 35 IU can set a dose limited to the 20 IU that are still available. The user is aware that only 20 IU can be set and dispensed and dispenses the remaining amount of medication required by the therapy, in this case 15 IU, from either a full cartridge inserted in a reusable device or 15 IU from a new and filled disposable device.

For a pharmaceutical company it can be advantageous that only a part of the nominal volume of the cartridge is dispensed during a single and/or repeated injection(s), either for a commercial product or during the development phase including clinical trials. For example if a cartridge contains 50 IU of a medication, only 30 IU can be dispensed in total and 20 IU cannot be dispensed. During the development and cumbersome and costly regulatory approval of a medication, it can be an advantage to have the approval only for the 50IU cartridge, which represents the main target group of patients. Developing a different formulation with a lower concentration or a lower fill volume as part of lifecycle management or for a new indication could be of less interest for the company, especially if the specific target group for the new formulation or fill volume is much smaller. For example if the main target group is for adults and the specific target group is for pediatric dosing applications. Thus using the same 50IU cartridge as for the main target group but restricting the amount of medication that can be dispensed to a lower, dispensable volume from the total volume (e.g. 30 IU) is beneficial and opens up therapeutic options to patients that would normally not benefit from the treatment. Thus from the nominal volume of a cartridge only a part, the dispensable volume is dispensed and a residual volume remains in the cartridge. For such therapeutic applications it would be desirable to have a dose limiting mechanism, which restricts the deliverable dose to a dispensable volume from the nominal volume and ensures that a residual volume of medication remains in the container and cannot be dispensed.

Other dosing devices are proposed in the state of the art, which prevent setting a of a dose which exceeds the amount of product present in the cartridge.

In EP2379138, a stop last dose for an injection pen is described where a restrictor element threadedly engages a piston rod to prevent setting of a dose that exceeds the amount of medication present in the cartridge. During setting of a dose, a dose-setting element is rotated and the restrictor element is slaved such that the restrictor element rotates and moves in the proximal direction with respect to the housing towards a counter stop element present on the piston rod. During dose delivery, the piston rod and the restrictor element both co-rotate, and advance in the distal direction with respect to the housing thus keeping the distance between the restrictor element and the counter stop element constant. During repeated dose setting and dose delivery, the restrictor element moves proximal with respect to the piston rod towards the counter stop until rotation of the restrictor element is prevented once the restrictor element engages the counter stop element and no further dose can be set. The restrictor element runs over the full length of the thread of the piston rod which prevents a compact arrangement of the dose limiter for the injection device and furthermore a single thread is used which prevents a gearing up/down movement of the restrictor element during dose setting.

In EP 2 829 292, a dose limiter is presented for an injection device having a sliding, e.g. non-rotating piston rod. The piston rod is axially guided through a passage of the housing preventing rotation. During dose delivery, the piston rod is driven by a rotation member that is threadedly engaged with the thread of the piston rod. During dose setting, a dose setting member is rotated and screwed outwards of the proximal end of the housing and during dose delivery, the dose setting member is returned into the housing and the rotation of the dose setting member is coupled to the rotation member. During dose setting a stop element that is also threadedly engaged with the external thread of the piston rod is rotationally coupled to the dose setting member and therefore rotates and screws distally onto the thread of the piston rod towards the rotation member thus lowering the axial distance between the stop element and the rotation member. During dose delivery, both the rotation member and the stop element rotate on the same thread of the piston rod and remain axially spaced at the distance set during dose setting while the piston rod slides in the direction for dose delivery. Further rotation of the rotation member is prevented as the stop element abuts the rotation member during a dose setting operation, thus limiting the total amount of doses that can be set from the device to the amount of medication present in the reservoir. This configuration is adapted to a sliding piston rod and uses the full thread length of the piston rod for the dose limiter as for dose delivery thus preventing a space saving arrangement of the components. Furthermore, a single pitch of the piston rod's thread is used for dose delivery and dose limiting without a gearing ratio for the stop element that could be beneficial for a space saving component arrangement.

A dose setting limiter for an injection pen with a track and a track follower is described in USRE41956. A dose setting member screws proximally out of the housing versus a non-rotating driver during a dose setting operation. A helical track is located at the outside of the cylindrical driver with a nut member acting as a track follower to count the accumulated doses. The nut member is threadedly engaged with the helical track of the driver and axially guided on the dose-setting member. During dose-setting, the nut member follows the rotations of the dose setting member and moves on the helical track of the non-rotating driver. During dose delivery, the drive member is rotationally coupled to the dose setting member and the position of the nut member on the helical track remains unchanged since there is no relative rotation between the driver and the dose setting member. After repeated dose deliveries, the nut member has moved in the proximal direction towards a fixed stop on the driver and once the nut member abuts the fixed stop at the end of the helical track further dose setting is prevented and no dose can be set that exceeds the amount of medication present in the cartridge. The length of the helical track thus corresponds to the total doses that can be spent form a cartridge. The nut member, or track follower runs on a helical track that differs from a thread present at the piston rod therefore allowing a different gearing for the piston rod and the track follower. This construction limits the options or needlessly complicates the device if different power packages are used to drive a piston rod. Furthermore dose limitation is based on the concept of one limiting part moving towards a fixed counter limiter. Furthermore, examples are shown with a two dimensional spiral track arrangement which is a disadvantage for a space saving arrangement in a pen type of device.

In EP1909870, a spring loaded injection device is described having a dose limiter limiting the accumulated doses that can be dispensed from the device. The doses are dispensed by advancement of a rotating piston rod that is in a threaded engagement with the housing. A dose is set by rotation of a dose setting member and a driver coupled to the dose setting member. The driver defines a passage for a dose limiter and during dose setting, the limiter is screwed on the external thread of the piston rod by the driver. The driver screws towards a stop position defined at the proximal end of the piston rod. During dose delivery, the driver and piston rod co-rotate to advance the piston rod and the axial position of the limiter with respect to the piston rod remains unchanged. Thus during repeated dose setting and deliveries the limiter screws towards the fixed stop at the end of the piston rod and prevents the setting of a dose that is above the amount of medication present in the cartridge. In this configuration, the limiter runs on the same thread of the piston rod as used for the piston rod advancement through the housing and therefore preventing a more compact design. Furthermore the driver is rotationally and axially fixed to the dose setting member therewith restricting the release mechanism of the spring loaded device to a mechanism located at the outer wall of the pen (side release) and not on the proximal end using a dose knob (top release).

WO2012125876 presents a stop last dose for an injection device having a non-rotating piston rod. During dose setting, a scale drum with a dose set knob is rotated and consequently moves proximally due to its threaded engagement with the housing. The scale drum rotates versus the housing and a driver is non-rotating during dose setting. A stop last dose is sandwiched between the rotating scale drum and the non-rotating driver and is shaped as an arc with an external thread that is driven by an internal thread of the scale drum. The arc is splined onto the outside of the tubular driver and slides distally during dose setting in the direction for expelling medication because of the relative rotation between the scale drum and the driver. The stop last dose arc moves towards a fixed stop present at the scale drum and prevents setting of a dose after an accrued amount of doses has been set. The stop last dose is driven by a thread segment on the inside of the scale drum and is designed for a non-rotating piston rod configuration, and, additionally is not suitable for other power packages for an autopen.

It is an object of the present invention to present an alternative dose setting mechanism for an injection device compared to the above mentioned mechanisms. Further objects of the invention are to provide a dose setting mechanism suitable to be used with different power packages to drive the piston rod for expelling medication. It is a further object of the present invention to reduce the size and complexity of the device and to offer options to dispense only a part of the nominal volume from a medicament container using a suitable limiting mechanism or assembly method for the device.

This object is solved with a drive and dosing device according to claim 1. Preferred embodiments are specified in the dependent claims, the description and the Figures.

### General description

The dose setting mechanism can be integrated into an injection device for delivering single or multiple doses of medication that each are set prior to dose delivery. The injection device can be a designed as a single use unit, e.g. a disposable device or used multiple times as a re-usable device. The injection device having the dose setting mechanism with the dose limiter delivers the doses from a container, for example a cartridge, containing the medicament. The cartridge comprises a tubular part that is closed with a septum at one end and the circular opening at the opposite end is closed by a stopper to form a compartment for containing the medicament. The septum at the distal end of the cartridge can be pierced by a needle or cannula for delivery of the medication to the patient. The cartridge is connected to the dose setting and delivery mechanism using a cartridge holder. At its proximal end the cartridge holder is connectable to the housing of the injection device. The connection between the housing and the cartridge holder is a releasable connection, for example a bayonet type of connector, for a reusable injection device or it is a permanent or semi-permanent connection, for example a snap-fit connection or an adhesive connection for a disposable or semi-disposable device. The cartridge holder has a tubular shape to encompass the cartridge at the opening from the proximal end. The cartridge holder has a connection site at its distal end for connecting a needle hub with a hollow needle to the cartridge holder. The hollow needle or cannula goes through the needle hub and after piercing the septum of the cartridge connects the inner volume of the cartridge via the needle to the patient.

The tubular housing has a means for viewing the current dose that has been set or corrected, for example a dose scale or an electronic display. The viewing means is, for example, a viewing window with or without a magnifying means. At the distal end of the housing, the cartridge holder is connected to the housing whereas a means for setting or correcting a dose, e.g. a dose-setting element, is located towards the proximal end of the housing. The user can set a dose by rotating and/or axially moving the dose setting element and viewing the dose set from the viewing means. Once a dose has been set, the user can dispense the dose by pressing an actuation button that is located on the housing or on the dose setting element. The actuation element can be located at the outside of the tubular wall of the housing but, preferably, is located at the proximal end of the housing or it is partially enclosed by the dose setting element. The user actuates the device by pressing the actuation button from a non-actuated into an actuated position. The actuation button can actuate the drive and dosing mechanism and finally drive a piston rod in the distal direction and move the stopper in the cartridge accordingly to expel medication from the device. Actuation of the device by pressing the actuation button releases potential energy from one or more of the following power packages such as a spring, for example a compression spring, a spiral spring, a helical spring, a coil spring, an extension spring or a torsion spring. The spring can have a single coil or have multiple coils or have a guide pin for guiding the spring forces. Alternatively, the potential energy is released from two separate springs which are coupled to each other in a series or parallel type of arrangement. The coupling can be a direct coupling between the two spring coils or via a third coupling member. The coupling between the two springs can be a permanent coupling or a releasable coupling. The coils of the two springs can have an identical diameter for a series type of arrangement or they are coaxially organized having different diameters. The kinetic energy needed for driving the injection can also be drawn from other sources such as an expansion of a gas that is compressed in a cartridge or gas that is released from initiating a chemical or electro-chemical reaction. Alternatively, the energy is released from an electronic source driving an electromotor or by volumetric changes of the electro-chemical source itself.

In the previous example, the dose setting element and/oractuation element are axially fixed with respect to the housing during a dose setting step and the energy for advancing the piston rod originates from a spring, or an electric source or a motor or an electro-chemical source. In another example, the actuation button and/or the dose setting element move in the proximal direction with respect to the housing during dose setting and the user returns the dose setting element and/or actuation element towards the housing during dose delivery. The piston rod of the device can also be advanced by returning the actuation button back from a proximal position achieved during dose setting back to the proximal end of the housing by manually pressing the actuation button. The axial displacement and/or rotation of the dose setting element or a driver is, in this case, transferred into the energy required to drive the piston rod distally into the cartridge. After the injection is finished and the dose expelled, the user releases the actuation button and the actuation button returns from the actuated position back to the non-actuated position.

The basic principle of the dose setting mechanism having a dose limiter is based on a first element that rotates during the setting of a dose with respect to a second element, which does not rotate during the setting of a dose. During the dose delivery step, the first element and the second element both rotate such that there is no relative rotation between the two elements. The first element rotates during the setting of a dose in a first direction and both the first and the second element rotate during dose delivery in a second direction, which is opposite to the first direction. If a dose is corrected during the dose setting process, then the first element rotates in the second direction without rotation of the second element. A first stop element is operationally, preferably rotationally coupled to the first element such that the rotation is transmitted from the first element to the first stop element. In a preferred embodiment, the first stop element axially moves during the dose setting process and is thus rotationally coupled but axially slidable with respect to the first element. The first stop element comprises a first limiter, which follows the axial and/or rotational movements of the first stop element and thus can be regarded functionally as an integral part of the first stop element. A second stop element is rotationally coupled to the second element such that rotations of the second element are transferred to the second stop element. The second stop element comprises a second limiter and the second limiter is designed such that it follows the axial and rotational movements of the second stop element. The limiter is directly provided on the stop element or it is, for example, inserted as a separate part but functionally can be regarded as a single unit. The first limiter and second limiter are configured to prevent relative rotation between the first and second element in one rotation direction when the first and second limiter are brought into engagement but allow for said relative rotation in a second direction which is opposite to the first rotation direction.

The first element, or drive sleeve can be rotationally coupled to the dose setting element such that a rotation of the dose setting element is transferred to the first element. Rotation of the dose setting element also operates a dose scale drum such that rotations of the dose setting element are transferred to the dose scale drum. The dosage currently set can be viewed through the viewing window of the housing so that the patient knows and is aware of the dosage selected from the device. In one embodiment, the dose scale drum is rotationally coupled to the first element and rotations and/or translations of the dose setting element are transmitted to the scale drum. The dose scale drum axially moves and/or rotates in the housing of the device and the axial movements or rotations are halted by axial or radial stops present in the housing thus defining the minimum (or zero dose) and the maximum dose that can be selected for a single injection. As described above, the first stop element moves axially during dose setting and thus its axial movements can be restricted by axial stops present in the dose setting mechanism, preferably within or located on the housing, and therefore constitutes an alternative manner to restrict the setting of a an individual dose between a maximum and minimum value.

The rotational coupling or decoupling of the first and second element is governed via a first coupling structure. Preferably, the first coupling structure is open during dose setting and closed during dose delivery but for certain applications it can be an advantage to have the opposite e.g. a closed first coupling structure during the dose setting process and an open coupling during dose delivery. The purpose of the first coupling structure is to operationally couple or decouple the first element to the second element such that it enables that the first and second element are in a rotationally locked or unlocked position with respect to each other depending on the actuation of the first coupling. The first coupling can be brought from the decoupled to the coupled position by actuation of the actuation element. Once the actuation element is released the first coupling is brought back into its original position, in the first example described above the coupling is decoupled from the coupled into the decoupled position.

The first element is coupled to the dose setting element via a second coupling structure, which rotationally couples the first element to the dose setting element when the second coupling is in the coupled state. The second coupling can be brought from the coupled into the decoupled state by pressing the actuation element of the device and therewith rotationally decoupling the first element from the dose setting element. In an alternative embodiment the coupling is reversed e.g. when the actuation button is not actuated the second coupling is decoupled and brought into a coupled state by pressing the actuation element.

The power package or spring which releases its potential energy upon activation for conversion into kinetic energy, is functionally located between the first element and the actuation element. For example, a wound-up spiral spring is on one end attached to the first element and on the other end on the actuation element. Either the spring is already preloaded or prestrained, and therewith putting the first element and actuation element under a permanent torsional tension, or the spring is loaded during each dose setting step by rotating the dose setting element. The actuation element is axially guided but rotationally locked to the dose setting element such that rotations of the latter are also transmitted to the actuation element. A linear guidance or fifth guiding connection between the actuation element and the dose setting member ensures that rotations are transmitted but that axial movements required for the actuation or the return-button process are allowed. The actuation button can therefore be moved from the non-actuated into the actuated position in the fifth linear guidance structure. The actuation button is preferably pressed against the force of a resilient spring which ensures that the actuation button returns to the non-actuated position if the actuation button is not pressed.

The actuation button and dose setting member are rotationally decoupled from the housing during the setting of a dose and are rotationally coupled to the housing in at least one rotation direction during the dose delivery process. For that purpose, a third coupling structure exists between the dose-setting member and/or the actuation element and the housing and the third coupling structure is in the decoupled position during dose setting and in the coupled position during dose delivery. The third coupling structure can be designed as a bidirectional coupling, e.g. it prevents relative rotations between the dose setting member and the housing in both directions but, alternatively, the third coupling structure is a unidirectional coupling or ratchet type of arrangement. In the latter case, the relative rotations between the housing and the dose setting member are prevented which ensure for a correct torque transmission, particularly from the spring to the drive member.

Depending on the coupling-decoupling sequence of the first, the second and the third coupling, the torque or force stored in, for example, the drive spring is released and enables the first element or drive sleeve to rotate. Several options are available for the coupling and decoupling sequences upon activation of the actuation element and as an example, the following sequence is presented for illustrative purposes. For example, the first and third coupling structures are open or decoupled during dose setting whereas the second coupling structure is in the coupled state such that the torque or spring force from the one end of the, for example, drive spring that is connected to the actuation element is guided to the dose setting element via the fifth guidance. The torque exerted from the other end of the spring to the first element is guided back to the dose setting element via the second coupling which is in the coupled state. Therewith the energy stored in the, for example, drive spring is in a closed loop and not released during dose setting. During actuation of the actuation element, the first coupling is brought into engagement and therewith coupling the first element to the second element. Subsequently, the third coupling structure is closed and therewith the actuation element-dose setting element are coupled to the housing and finally the second coupling structure is decoupled, decoupling the first element from the dose setting element. As a result, the potential energy stored in the spring is functionally positioned between the housing, via the assembly of dose setting member- actuation element, via the first element to the second element. Subsequent rotation of the second element or coupling sleeve ensures that the piston rod is advanced in the cartridge due to a thread connection of the piston rod, for example between the piston rod and the housing. The potential energy stored in the power package is thus transferred into kinetic energy once the piston rod advances and moves the stopper in the cartridge to expel a medicament.

The first stop element is guided during the dose setting process and dose delivery process by a first guidance or guiding connection to ensure that rotations of the first element are transmitted to the first stop element. During the dose setting process, the second coupling between the first element and the dose setting element is in the coupled state and therefore the rotations of the dose setting element lead to a rotation of the first stop element. During dose delivery, the second coupling is decoupled and the first and second element rotate in a direction that is opposite to the dose setting direction. Also during dose delivery the first stop element follows the rotations of the first element due to the first linear guiding connection. The guiding connection or first guidance ensures that rotations of the first element are transferred to the first stop element but allows for relative axial displacements between the two elements. The first stop element has a thread segment that ensures that the rotations of the first stop element result in an axial displacement. The thread segment is an integral part of the first stop element, thus can be viewed as a single part. The thread segment of the first stop element is in a threaded engagement with one of at least one thread element and this at least one counter thread can be located on a part fixed to the housing or the housing itself, or it is located on a part that is not fixed to the housing, for example the scale drum or on a second stop element that will be described below, or it is located on a part that does not rotate during dose setting. The thread segment of the first stop element ensures that upon rotation, the first stop element axially displaces with respect to the housing or housing part. During dose setting, when the dose setting member and first element are rotated in a first direction, the first stop element moves in one direction, preferably parallel to the longitudinal axis of the injection device. During dose correction, the first element rotates in a second direction which is opposite to the first direction and the first stop element rotates and moves axially in a second direction which is opposite to the first direction. During the dose setting / correction process the first element is rotationally decoupled from the second element and therewith the second stop element that will be described later does not move along the longitudinal axis.

The first stop element rotates and moves axially during dose setting with respect to the housing from a first position to a second position. The axial distance between the two positions corresponds to the amount of dosage set. In an embodiment, the first position is a proximal position and the second position is a distal position with respect to the housing. The opposite arrangement e.g. moving from a first distal position to a second proximal position can also be realized by selecting, for example inverting the threaded engagement of the first stop element to the housing or a housing part. During dose delivery, the first stop element returns from the second position back to the first position. During repeated dose settings and dose deliveries, the first stop element returns each time back to the same first position with respect to the housing, e.g. the first stop element reciprocates along the longitudinal axis of the injection device whereby each time the second position is variable and corresponds to the dosage set and the first position is each time identical.

The second stop element that does not rotate during the dose setting process with respect to the housing is operatively coupled to the first stop element during dose delivery, thus both rotate in the same direction during dose delivery. The second stop element is in a threaded engagement with one of at least one thread element that is part of the housing or a part fixed to the housing, or on a part that does not rotate during dose setting. The first and second stop element can use the same threading or thread element for rotational engagement and axial displacement, or the first and the second stop element each have their own and separate threadings or thread elements for engagement with the corresponding thread segments of the stop elements. The threadings or the thread elements in terms of slope or pitch can be identical but also embodiments can be envisaged where each stop element has its specific thread pitch or slope. In the latter case each of the first and second stop elements has a specific threaded engagement with a part of the housing. The threaded engagement can be designed to be a single threaded engagement or a multiple threaded, for example a double or triple threaded engagement. During dose setting, the second stop element does not rotate or axially move since the part is rotationally decoupled from the rotating first element. During dose delivery, the first and second elements co-rotate and the second stop element is slaved such that it rotates and due to the threaded engagement also axially moves with respect to the housing. The first and second stop elements both can move in the same, for example proximal direction during dose delivery, which corresponds to the amount of dosage selected. Thus during dose setting the first stop element axially moves from a first to a second position, the distance corresponding to the dosage set whereas the second stop element does not move during dose setting. During dose delivery both the first and the second stop elements axially move with respect to the housing along a distance that is equal or proportional to the distance between the first and second position of the first stop element during the dose setting procedure.

The first stop element has a first limiter and the second stop element has a second limiter. The limiters are preferably directly connected or an integral part of the first and second stop elements respectively. The first and second limiters, as being directly connected to the first and second stop elements, perform the same axial and/or rotational movements as the first and second stop elements. Thus during dose setting the first limiter rotates and axially moves from a first position to a second position whereas the second limiter does not move. The axial or angular distance between the first and second limiter is preferably reduced during the dose setting process and the axial or angular distance between the first and second limiter remains constant during dose delivery. Thus during repeated dose setting and dose delivery, the axial or angular distance between the two limiters is reduced. The initial axial or angular distance between the two limiters can be programmed to represent an amount of medication present in the reservoir. During repeated dose setting and dispensing steps, the distance between the limiters is reduced until the two limiters abut and prevent the setting of a dose that goes beyond the amount initially programmed. The initial amount of medication programmed to be dispensed from the cartridge or reservoir can be the nominal volume present in the reservoir but can also be the dispensable volume from the reservoir, which is less than the nominal volume of medication in the reservoir. In the latter case, the setting of a dose which goes beyond the dispensable amount of medication is prevented and a residual amount of medication that cannot be dispensed remains in the cartridge. The amount initially programmed to be dispensed is thus a predetermined amount of medication which cannot be set after the accumulated dosages have been set and dispensed. Once the first and second limiter abut or are in engagement, no dosage setting by rotating the dose setting member in the first direction above the predetermined amount is allowed whereas the set dose can still be corrected by rotation of the dose setting member in the second direction. During dose delivery, the set dose can be dispensed and the first element is rotated together with the second element in the second rotation direction.

The second stop element moves during repeated dose deliveries in an axial direction, preferably the proximal direction. Thus whereas the first stop element performs a pendulum type of back and forth movement, the second stop element moves unidirectionally. After repeated dose settings and deliveries the engagement of the first and second limiters prevents the setting of a dose which goes beyond a predetermined amount.

The first and second limiters are designed to act as a radial or axial stop and/or as a radial or axial counterstop. The stop and/or counterstop each comprise a stop surface or counterstop surface and these surfaces contact each other when the stop and counterstop are in abutment and the overlap between the stop surface and the counter stop surface defines a contact area between the stop and counter stop. The contact stress between the stop and counterstop is defined by the contact force divided by the contact area. Once the stop and counterstop are engaged and/or abut due to an axial or radial approach, the stop-counterstop arrangement can act unidirectionally therewith preventing the relative movement between the first and second limiter in the first direction only. In the other direction, which is opposite to the first direction, relative movements are allowed. As an alternative, also bidirectional movements can be prevented once the first and second limiters are in an engagement. The two limiters can have a saw tooth type of structure for the stop and counterstop surfaces or have a radial stop mechanism or comprisepins that interlock upon abutment. The limiters can have a rigid or flexible resilient type of nature or are part of a ratchet type of mechanism. The engagement between the two limiters, e.g., the stop can be reversible or irreversible. For an irreversible stop the two limiters can have a resilient arms that interlock or snap into each other to form a bidirectional stop. Alternatively, the two limiters can engage in a friction fit to form a reversible or irreversible stop. For a friction fit, the two materials of the two limiters are the same or can be different, each having their own frictional coefficient. During the approach or engagement of the two limiters one or both of the first and second limiters can plastically deform which can be enabled by the selection of the two plastic materials of the limiter, each having the same or different material properties like modulus and strength. As an example, for illustrative purposes only the first and second limiter can be made from a polymeric material, for example ABS, Polyoxymethylene POM, Polyethylene (HDPE, LDPE, LLDPE, UHMWPE), polypropylene (PP), Polysulfone, PPE, Polystyrene, Polycarbonates, PPSU etc. Alternatively one or both limiters are made from a metal.

In the stopping position when the first and second limiter of the first and second stop elements are engaged, a dial up torque is transferred from the dose setting element to the housing in a cascade type of arrangement. As an example, for illustrative purposes only, such a cascade can be a force or torque from the dose setting element to the first element, and from the first element to the first stop element, and from the first stop element to the second stop element via the contact area between the two abutting limiters, and from the second limiter to the second element, and from the second element to the housing via a third element or reverse lock sleeve that will be discussed later.. The linear and/or angular advancement of the first and second limiters depends on the threaded engagement of the first and second stop elements, for example to the housing. For a compact and space saving design it can be an advantage to have a threaded engagement with a low slope or pitch, e.g. the first and/or second stop element advances over a relatively small axial distance per rotation. As a result, the contact area between the first and second limiters upon engagement is also relatively small which can lead to high contact stresses or, eventually, plastic deformation of the contact area. As an alternative, the plastic materials can be enforced, for example with glass fibers such that the higher contact stresses during engagement and over-torqueing are compensated for by an increased stiffness and strength. As an alternative, also the contact area between the two limiters can be increased without jeopardizing the space saving arrangement requiring a relatively low pitch of the threaded engagement. For this, one or both of the first and second stop elements run on a thread with a variable pitch which has a low value or slope during the repeated dose settings, e.g. before the last dose has been set, but has a high pitch or slope at the end of the thread such that during the setting of the last dose the first and/or second limiter axially move over a larger distance. The increased axial movement during the setting of the last dose results in an increased overlap area between the first and second limiters and thus decreases the contact stress upon abutment. With such a variable pitch design, more options are open to combine a space saving arrangement with a cost saving choice of materials since expensive engineering polymers having a high strength and stiffness can be avoided. Alternatively, the first and second stop elements can be provided with more than one limiter, for example the first and/or stop element can be provided with two or three limiters and each limiter can act as a stop or counterstop. Thus in the stopping position the more than one limiter of the first stop element can be in engagement with one or more of the limiters of the second stop element.

The dose scale drum is operationally coupled to the dose setting member such that rotations of the dose setting member also result in rotations of the scale drum. A second guiding connection or guidance is, preferably, present between the first stop element and the scale drum. Alternatively, this guidance is located between the first element and the scale drum. The dose scale drum in one example is threadedly engaged with the housing and rotation of the scale drum axially shifts the scale drum during dose setting. In a preferred embodiment, the scale drum axially moves in the distal direction during dose setting and moves in the proximal direction during dose correction. The minimum and maximum doses that can be set for a single dose to be selected e.g. 0 IU and, for example 60 IU, are governed by axial or radial dose stops designed at the scale drum, which abut counter dose stops present, for example, at the housing such that the setting of a single dose above 601U by rotating the dose setting member in one direction is prevented. Setting of a dose below the minimum dose of 0IU by rotation of the dose setting member in the second direction is prevented by another axial or radial dose stop of the housing or housing part. The scale drum is, preferably of a tubular shape with on one side, preferably inside, a second linear guide component, which ensures that rotations of the first stop element result in rotation of the scale drum but allowing also for axial movements. The outside of the scale drum preferably has a groove or thread segment that is in engagement with a threading of the housing. Additionally, numbers corresponding to a dose are printed on the outside surface of the scale drum along a helically shaped curve such that the numbers can be viewed through the viewing window of the housing. As an alternative, the dose scale drum is threadedly engaged with another element present in the housing, for example the first and/or second stop element.

The second element or coupling sleeve is preferably shaped as a sleeve with a longitudinal axis preferably coaxially with the main axis of the housing. The second element is axially engaged to the housing and therewith preventing axial movement relative to the housing but allowing rotations. The engagement between the housing and the second element can be a bearing type of engagement between a protruding rim on the coupling sleeve and a groove in the housing or housing part. The engagement between the second element and the housing can be designed to allow for bidirectional rotations or for a rotation in one direction only e.g. by a ratchet means. In the latter case, the allowed rotation corresponds to the rotation direction required for dose delivery. The second element has a second coupling structure, preferably located at the proximal end of the coupling sleeve such thatreversible coupling/decoupling to the first element having a first coupling structure is enabled. The first and second coupling structures are designed such that the in the decoupled state relative rotations between the first and second element are allowed whereas the first and second element are rotationally coupled once the first and second coupling structures are engaged. The first and second coupling structures are brought in the coupled state by pressing an actuation element. Upon release of the actuation element, a resilient member forces the first and second coupling structures into the decoupled state. As an alternative, the switching of the actuation member between the decoupled and coupled state can be done manually without the need of a resilient member. The sleeve like second element encloses a delivery means, for example a piston rod, required to advance the stopper in the cartridge to expel medication from the device. The second element is operatively coupled to the delivery means for delivery of the medication and there are in principle two options for this engagement to transfer a rotational movement of the second element into an axial advancement of the delivery means. In the first option, the sleeve like second element is keyed to the delivery means which is shaped as a longitudinal rod having one or more keyways running along the longitudinal axis that match with one or more keys of the second element. The engagement is such that there are axial movements allowed between the second element and the delivery means whereas relative rotational movements are prevented. A rotation of the second element results in a rotation of the delivery means which, through a threaded engagement, for example to the housing or a housing part, can screw and advance in the distal direction. In the second option, there is a threaded engagement between the outside of the piston rod and the inside of the coupling sleeve. In such an arrangement the piston rod also has at least one longitudinal keyway,or rim, but this keyway or rim is keyed to the housing or housing part preventing relative rotations between the housing and the piston rod. Rotation of the second element in the second direction in this arrangement slides the piston rod into the direction for dose delivery.

The second stop element having the second limiter is operatively coupled to the second element or coupling sleeve such that a rotation of the second element results in a rotation of the second stop sleeve. The operative couplings or linear guidances allow for relative axial movements between the second stop sleeve and the other coupling partner but prevent relative rotational movements. The second stop sleeve can be directly coupled to the second element, for example via a protrusion of the second stop sleeve that is keyed to a groove in the second element or vice versa. In a preferred embodiment, the second stop sleeve is operatively coupled to the second element via at least a third element or reverse lock sleeve. Since the rotational movements of the second element need to be transferred without limiting the axial movements, at least two linear guidance structures are required between the second element and the third element and the third element and the second stop sleeve. The linear guidances are each composed of keys that match longitudinal keyways and thus the second element, third element and the second stop element are in a telescopic type of arrangement. In a preferred embodiment, the third element is coaxially arranged around the second element and sandwiched between the second stop sleeve and the coupling sleeve.

The third element or reverse lock sleeve is operatively coupled between the second element and the housing. The reverse lock sleeve or third element ensures that the second element can rotate in one direction only. The allowed rotation direction, for example the second rotation direction, is the rotation direction required for dose delivery and advancement of the piston rod. The opposite direction or first rotation direction is the rotation direction for increasing a dose during dose setting. Any torque that acts on the drive system in the first direction and potentially also on the second element, is absorbed by the reverse lock element. Additionally, axial forces acting upon the delivery means in the proximal direction opposite to the delivery direction need to be compensated for by selective prevention of some of the rotating components, for example rotation of the second element of the drive train in the first rotation direction to prevent any unwanted back-drive of the delivery means which would lead to a reduced accuracy. The reverse lock element or reverse lock sleeve has, for example, a tubular shape with an axial or radial ratchet system or a two-way clutch, preferably in combination with the housing or a housing part such that the reverse lock sleeve can only rotate in the second direction when the ratchet system is biased by a biasing means. The reverse lock sleeve for example has at least one asymmetric saw tooth structure present at one of the two ends of the sleeve, which can be in an engagement with a complementary asymmetric saw tooth structure present on the housing or a housing part. The two saw tooth structures compose a unidirectional ratchet system that prevent rotation in one of the two rotation directions. In the other of the two rotation directions the ratchet system allows for relative rotations and produces a clicking sound once the saw teeth ratchet over each other. The allowed rotation direction, for example the second rotation direction corresponds to the rotation direction for dose delivery and the ratchet system thus acts as a dose delivery clicker. The ratchet system preferably acts as a undirectional ratchet system preventing rotation of the second element in the first or dial up rotation direction both during dose setting and dose delivery.

The ratchet system is designed such that parasitic or frictional torques in the second or dial down rotation direction are compensated for by a specific resilient torque of the unidirectional ratchet thus preventing rotation of the second element in the second rotation direction during dose setting. During dose delivery the rotational torque of the drive train in the second rotation direction exceeds this specific resilient torque and the second element rotates in the second rotation direction.

The reverse lock sleeve can be rotationally coupled but axially slideable connected to the second element using the third linear guidance. For example on the inside of the reverse lock sleeve at least one protrusion can be keyed to at least one linear groove present on the second element. The protrusion to groove arrangement can also be reversed, e.g. at least one longitudinal groove on the reverse lock sleeve can match one protrusion present on the second element. Thus a rotation of the second element is transferred to the reverse lock sleeve and thus produces the audible delivery clicks. The third linear guidance also prevents the reverse rotation of the second element in the first direction because of the unidirectional ratchet system. The reverse lock sleeve serves at least a dual purpose.

In a preferred embodiment the reverse lock sleeve is concentrically arranged around the second element and keyed to the second element by the third linear guidance. The second stop element is keyed to the reverse lock sleeve by a fourth linear guidance. The fourth linear guidance allows for axial movement but prevents relative rotation between the reverse lock sleeve and second stop element. The fourth linear guidance in combination with the ratchet system of the reverse lock sleeve prevents that the second stop element rotates in the first direction, the dose setting direction. On the other hand, the fourth linear guidance to the reverse lock sleeve and the third linear guidance to the second element ensures that rotations of the second element in the second direction during dose delivery are transferred to the second stop sleeve. The rotations of the second stop sleeve are transformed in axial movements due to the threaded engagement of a thread segment of the second stop element with the housing or the housing part.

The reverse lock element or reverse lock sleeve is preferably concentrically arranged around the second element or coupling sleeve and a biasing means biases the ratchet system to prevent rotation in one rotation direction. The biasing means can be, for example, a spring made from a metal or a polymer. The biasing means is a separate part or is integrated into the reverse lock sleeve as one or moreresilient structures e.g. arms. The force pushing the ratchet system into the engaged position must be beared, for example the biasing means is beared on the housing or on the second element or on the first element or on the first stop element. Thus, in a preferred embodiment a spring is located between the reverse lock sleeve and a rim or protrusion expanding outwardly from the second element.

The user normally sets a desired dose of medication and grabs the dose setting element and rotates it until the desired dose is displayed in the viewing window. The dose setting is in discrete steps - for example each step corresponds to 1 IU or 0.5 IU and it is desirable that an audible means signals each dose step independent on whether this is a dial up or dial down operation. The dose setting mechanism with the dose limiter has a bidirectional ratchet system designed to notify each dose step to the user and is linked to the scale printed on the dose scale to show the actual amount of dose that has been set. The dose ratchet system or dose setting click system is preferably functionally located between the first and second element or as an alternative between the dose setting element and the housing. During dose setting, the first element rotates relative to the second element and the relative rotation can be utilized for the dose setting click. During dose delivery the first and second element are rotationally coupled to each other and the absence of relative rotations decommissions the dose setting ratchet system. The relative rotations present between the first and second element during dose setting are, due to the first linear guidance system also present between the first stop element and the second element. Due to the third and fourth linear guidance structures there are also relative rotations between the first element and the second stop element during dose setting. The guidance systems also ensure a relative rotation between the first stop element and second stop element during dose setting. As a consequence there are numerous options for designing an adequate dose setting clicker in the current invention and the following example is presented for illustrative purposes only. In one example, the dose setting/adjustment click is present on the first stop element or the first element as a flexible arm that ratchets over a number of grooves which are part of a second coupling structure present on the second element. The flexible arm has a ratchet element on the one end and the ratchet element can ratchet over the second coupling structure once there are relative rotations between the first element and the second element. The ratchet system is designed such that it allows for bidirectional rotations and the ratchet systems produces audible clicks. The ratchet arm with the ratchet element latches in discrete positions that correspond to discrete units set by the dose setting element. The ratchet arm is preferentially oriented in the circumferential direction of the housing, e.g. perpendicular to the longitudinal axis. In another example, the dose setting clicker is functionally located between the dose setting member and the housing. A resilient arm present on the dose setting member can flex on a coupling structure, for example the fifth coupling structure to produce the desired dose setting increments and audible clicks.

During dose setting, the scale drum rotates and moves axially away from the zero dose position until a specific dose has been set which is below or equal to the maximum settable dose as defined by a maximum dose postion of the scale drum. During dose delivery, the spring energy is released and the first and second elements rotate in the second direction and the scale drum rotates and moves back towards the zero dose postion. The dosing stops, e.g. the first and second elements stop rotating once the scale drum has reached the stop zero postion.

During each dose setting and dose delivery process the axial distance or rotational angle between the first and second limiters of the first and second stop element is reduced. The initial axial distance or rotational angle between the first and second limiter defines a predetermined amount of medication that can be dispensed from the reservoir. Once the first and second limiters of the first and second stop elements engage, they prevent the setting of a dose which would result in that the total sum of accumulated doses would go beyond the predetermined amount of medication to be dispensed from the reservoir. Further rotation of the dose setting element in the first direction is prevented but the last dose can still be injected since rotation of the first element in the second direction is allowed by the limiting mechanism. The predetermined amount of medication to be dispensed from the reservoir can be the nominal volume contained in the reservoir but can also be selected and programmed to be below the nominal volume, e.g. the dispensable volume. If the predetermined amount of medication that can be dispensed from the reservoir corresponds to the dispensable volume, then a residual volume remains in the reservoir when the first and second limiters are engaged.

In one embodiment, the dose setting and limiting mechanism is programmed by using a specific method for assembling the dose limiting mechanism. The initial axial or rotational distance between the first and second limiters of the first and second stop element corresponds to a predetermined amount or volume that can be dispensed from the reservoir. By selecting a specific distance or rotational angle, for example by the initial position of the first and second stop elements on the internal thread segment of the housing or housing part, the predetermined amount of medication to be dispensed from the reservoir can be programmed.

A dose setting mechanism for an injection device for injecting a dose of medication from a reservoir comprising a housing with a distal and a proximal end, and having a longitudinal axis whereby the reservoir is located near or at the distal end of the housing. Furthermore, the dose setting mechanism has a dose-setting element which is rotatable with respect to the housing in a first direction for setting a dose of medication and a first element operatively coupled with the dose-setting element and which is rotated with respect to the housing in the first direction during dose setting, whereby the first element rotates in a second rotation direction which is opposite to the first rotation direction during dose delivery. The dose setting mechanism comprising a second element that does not rotate during dose setting with respect to the housing and which is coupled to the first element during dose delivery for rotation in the second direction. The mechanism is characterized by a first stop element with a first limiter, the first stop element being threadedly engaged with one of at least one thread element, the one of at least one thread element is provided on a housing part or on a part that does not rotate relative to the housing during dose setting. The first stop element is operatively engaged with the first element such that the first stop element rotates in the first direction during dose setting and moves along the longitudinal axis from a first position to a second position due to the threaded engagement with the one of at least one thread element, and wherein the first stop element rotates in the second direction during dose delivery and moves from the second position back to the first position. The dose setting and limiting mechanism further comprises a second stop element with a second limiter, the second stop element being threadedly engaged with one of the at least one thread element and which is operatively engaged to follow rotations of the second element and which moves along the longitudinal axis during dose delivery due to the threaded engagement with the one of the least one thread element, and wherein the setting of a dose which exceeds a predetermined amount of medication present in the reservoir is prevented when the first and second limiters are engaged.

The dose setting mechanism comprising a third element which is operatively coupled between the second element and the housing such that the second element is rotatable with respect to the housing in the second direction and non-rotatable with respect to the housing in the first direction. Preferably, the third element comprises a one-way ratchet or a two way clutch.

The dose setting mechanism wherein the axial or angular distance between the first limiter of the first stop element and the second limiter of the second stop element reduces during dose setting and remains constant during dose delivery. Preferably, the first stop element and the second stop element each have thread segments that engage or can engage with the same of the one of the at least one thread element. The first stop limiter is, preferably integrally formed on, or provided on the first stop element and designed as an axial or radial stop and/or counterstop, and wherein the second stop limiter is integrally formed on, or provided on the first second stop element and designed as an axial or radial stop and/or counterstop. Furthermore, the predetermined amount of medication present in the reservoir corresponds to the nominal volume of medication contained in the reservoir, or wherein the predetermined amount of medication present in the reservoir corresponds to a dispensable volume from the nominal volume, which is below the nominal volume of medication present in the reservoir. Preferably, the dose setting mechanism having the first and the second stop elements threadedly engaged with the same thread element of the one of at least one thread element, or wherein each of the first and the second stop elements is threadedly engaged with two separate thread elements of at least two thread elements provided on a housing part or on a part that does not rotate relative to the housing during dose setting. The two separate thread elements each have, preferably, an identical pitch or a different pitch and, preferably one of the two thread elements is located on one of the first stop element or second stop element. An injection device for injecting a dose of medication having the dose setting and limiting mechanism described above, wherein the second element is operatively engaged with a piston rod such that a rotation of the second element advances the piston rod for injecting a dose of medication. Preferably, the injection device has the second element splined to the piston rod such that the piston rod and the second element are in a non-rotatable but slidable engagement and wherein the piston rod has an external thread that is in engagement with an internal threading of the housing such that a rotation of the second element rotates and advances the piston rod for delivery of a dose of medication from the reservoir. Alternatively, the piston rod has an external thread that is threadedly engaged with the second element and wherein the piston rod is non-rotatable and axially slidable engaged with respect to the housing such that a rotation of the second element advances the piston rod for delivery of a dose of medication from the reservoir.

### Definitions

The total volume of a medicament container (for example a cartridge, pre-filled syringe or ampoule) represents the maximum volume of the container. The total volume is composed of a dead volume, which cannot be dispensed from the container even if the stopper in a cartridge has advanced to its end position and a nominal volume. The nominal volume is the volume of medication that in principle can be dispensed from the container. This nominal volume can be dispensed completely or there remains a residual volume, which is less than the nominal volume that is not dispensed from the container.

The distal direction is the direction, as seen from the longitudinal axis of the housing, which points towards the direction for dose delivery. The proximal direction is the opposite direction.

Rotational movements or positions have the longitudinal axis as an axis of rotation. Axial movements or postions are along or parallel to the longitudinal axis.

A thread element is an element having a threading which can be a single or a multiple threading. Such a thread element can be located inside, for example, the housing. The thread element can be an inside thread element or and outside thread element. An inside thread element is a thread element which threading is pointing towards the longitudinal axis of the device and an outside thread element points in the opposite direction, outwards of the device. A thread segment is a part of threading running over a certain angle which is typically below 360 degrees, preferably below 180 degrees.

### Legends to figures

- Figure 1:: Exploded view of an injection pen having the dose setting mechanism according to a first embodiment of the invention, initial state with full cartridge.
- Figure 2:: Cross section of the injection pen having the dose setting mechanism according to the first embodiment of the invention, prior to setting of a dose.
- Figure 3:: Cross section perpendicular to the view of Figure 2 for an injection pen according to the first embodiment, prior to setting of a dose.
- Figure 4:: Cross section of the injection pen having the dose setting mechanism according to the first embodiment of the invention, after setting an initial dose.
- Figure 5:: Cross section perpendicular to the view of Figure 4 of the injection pen having the dose setting mechanism according to the first embodiment of the invention, after setting an initial dose.
- Figure 6:: Cross section of the injection pen having the dose setting mechanism according to the first embodiment after setting a dose and actuation of the actuation element.
- Figure 7:: Cross section of the injection pen having the dose setting mechanism according to the first embodiment of the invention after dispensing a dose.
- Figure 8:: Cross section of the injection pen having the dose setting mechanism according to the first embodiment of the invention after dispensing the last dose.
- Figure 9:: Cross section of an injection pen having a dose setting mechanism according to a second embodiment of the present invention, having two separate internal thread elements on the housing for guiding the stop elements, initial state with full cartridge.
- Figure 10:: Cross section of an injection pen having the dose setting mechanism according to a third embodiment of the present invention, initial state with full cartridge, dose limiting programmed to expel a dispensable volume from the nominal volume.
- Figure 11:: Cross section of an injection pen having the dose setting mechanism according to a fourth embodiment of the present invention having two separate internal thread elements for guiding the stop elements one thread element is on the housing, the other thread element is on one of the two stop elements, initial state with full cartridge.
- Figure 12:: Arrangement of first and second stop elements according to a fifth embodiment.

### Detailed description of the invention

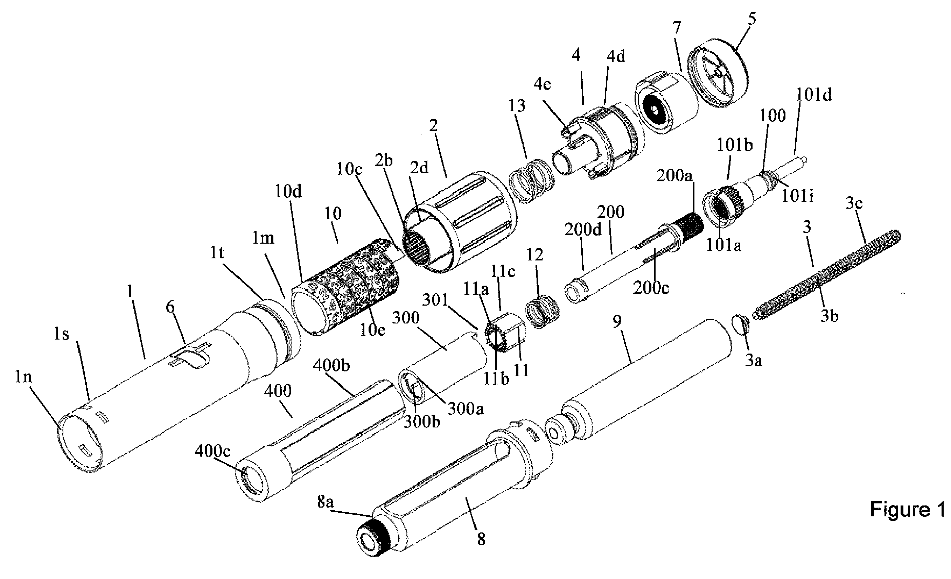

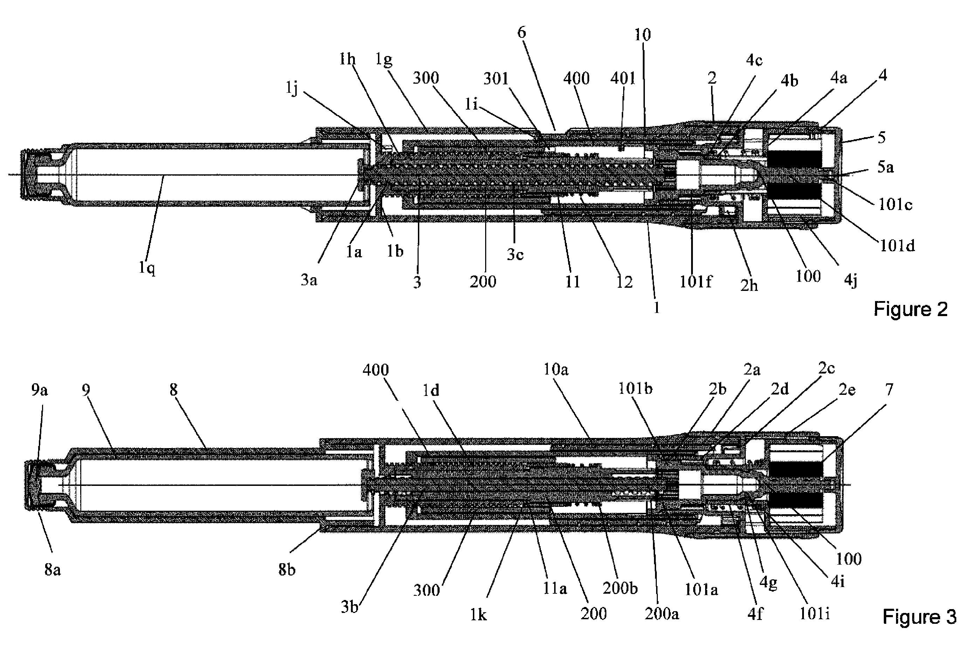

An exploded view of an injection pen having the dose setting mechanism according to a first embodiment of the invention is shown in Figure 1. The injection pen comprises a tubular housing (1) having a longitudinal axis (1q) with an outer housing or housing sleeve (1g, Figure 2). The tubular housing has a distal end designed for attaching a cartridge holder (8), for example, for a snap-fit connection (1s). The outer housing sleeve (1g) has a viewing window (6) for viewing the numerical values (10e) printed on a dose scale drum (10). On the proximal end, the housing ends in a circumferential rim (1m) for engaging a dose setting element (2) via a notch (1t). In the distal section of the housing, the outer housing sleeve (1g) has a circumferential rim (1b) pointing towards the inside of the housing for connecting an inner housing sleeve (1h) (Figure 2). The inner housing sleeve (1h) has a tubular shape with a certain length and the distal end of the inner housing sleeve is fixed to the rim (1 b), and the proximal end of the inner housing sleeve points towards the proximal end of the housing. An thread element or threading (1d, Figure 3) is located on the outside of the inner housing sleeve (1h) for engaging at least one of the first and/or second stop elements (400, 300). The rim (1b) also supports an internal housing threading (1a) for engaging an outer threading (3c) of a piston rod (3) as shown in Figure 2. The distal end (1 n) of the outer housing sleeve (1g) is designed to receive the cartridge holder (8) containing the cartridge (9). Furthermore, the outer housing sleeve (1g) has an internal thread or thread segment (1i) for engaging an outer threading (10a) of the scale drum (10).

The cartridge holder (8) has a tubular shape with a circumferential rim (8b) on the outside defining together with the distal rim (1 n) of the housing the end position of the cartridge once attached to the housing (1) as shown in Figures 1 and 2. The cartridge holder (8) has a needle connector (8a) at the distal end for connecting a needle hub to the cartridge holder. The needle connector (8a) can be used either for an axial click-on needle hub or for a screw-type of connection. The needle connector (8a) has a diameter that is below the main diameter of the tubular cartridge holder to receive and axially position a shoulder of a cartridge (9) that is contained in the holder. The cartridge (9) has a cylindrical shape that can fit into the cartridge holder (8) and has a pierceable septum (9a) at its distal end. The wall of the cartridge is made from glass or a polymer and is preferable transparent so the user can view the medication contained in the cartridge. At the proximal end, a piston or stopper (not shown in the Figures), preferably made from a rubber material closes the cartridge to form a compartment that can be filled with the medication.

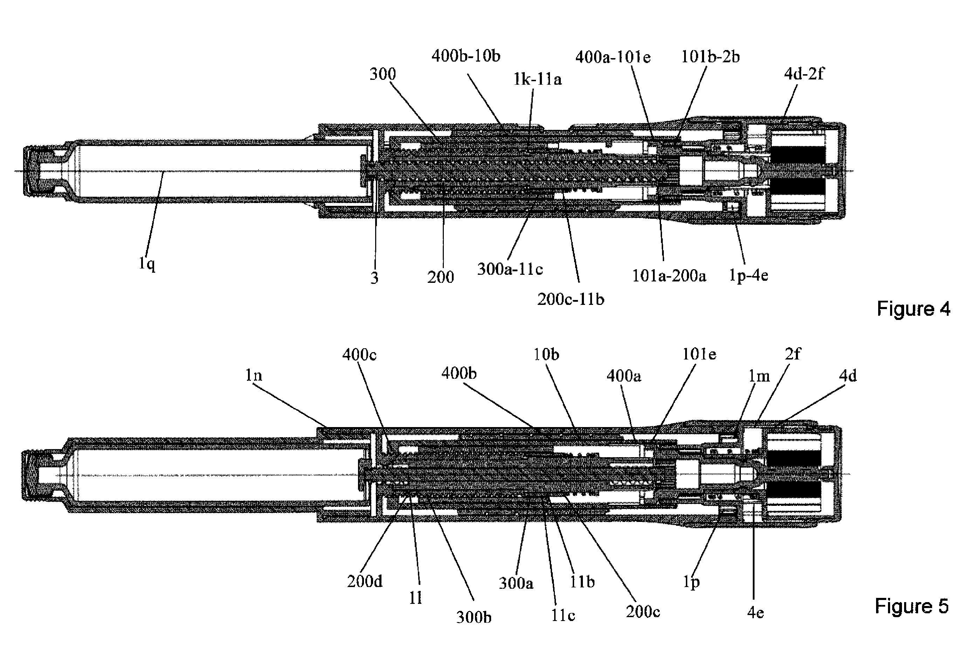

A dose setting element (2) is located at the proximal end of the housing (1) and is composed of an outer sleeve with a proximal rim section (2e) and a distal rim section (2a) that are connected by a circumferential rim (2c) that points towards the center of the device. The distal rim projection (2a) encloses the proximal rim (1m, Figure 5) of the housing and, preferably, the distal rim (2a) has a circumferential protrusion on the inside that matches a corresponding notch (1t) on the outside of the housing for an axially fixed but rotational free connection between the dose setting element (2) and the housing (1). The dose setting element (2) in this embodiment thus can rotate with respect to the housing without any axial movements during the dose setting process. An inner tubular sleeve (2d) is part of the dose setting element and connected to the inwardly protruding rim (2c). The proximal end of the inner sleeve (2d) is connected to rim (2c) and the distal end of the inner sleeve points in the distal direction of the device. A third coupling structure (2b) is designed to be part of the inner sleeve (2d), the third coupling structure (2b) builds together with a fourth coupling structure (101 b) of a first element (100) a second coupling (101 b-2b, Figure 4). The third coupling structure (2b) comprises, for example, linear and longitudinal grooves on the inside of the inner sleeve (2d) that can match corresponding grooves or notches on the first element (100) for the second coupling (101 b-2b) that is rotationally fixed in the coupled state and allows for relative rotations in the decoupled state. The proximal projection (2e) of the dose setting element (2) encloses a release button (4) that is located at the proximal end of the device. The release button or actuation element (4) is composed of a sleeve having an outer diameter that fits into the inner diameter of the proximal end (2e) of the dose setting element (2). The release button (4) is closed at the proximal end by a release button (5) which forms the outer surface that the user can press with his thumb for actuation of the actuation element. A fifth linear guidance (4d-2f, Figure 5) exists between the dose setting element (2) and the release button (4). Linear guiding elements such as grooves oriented parallel to the longitudinal axis (1q) are on the outside of the cylindrical release button (4) that match corresponding linear grooves or notches on the inside of the proximal rim (2e) of the dose setting element (2). The fifth linear guidance guides the release button (4) axially during the actuation process from the non actuated into the actuated position. The fifth guidance also prevents relative rotations and ensures that rotational torque is transmitted between the two elements both in the actuated and the non actuated position of the release button (4). The user of the device thus can grab the dose setting element (2) and rotate the dose setting element (2) with respect to the housing (1) for setting a dose and the release button (4) will follow the rotations of the dose setting element. In this embodiment the dose setting element will not axially move with respect to the housing but other options can be realised whereby the dose setting element upon rotation axially moves in the proximal direction with respect to the housing. The release button (4) is constructed as an outer sleeve (4j) and an inner sleeve (4f) connected by a sleeve connector (4i). The inner sleeve (4f) has a smaller diameter than the outer sleeve (4j) and encloses a first element or drive sleeve (100). The inner sleeve (4f) has a rim shaped protrusion (4g) on the inside that matches a notch (101 i) on the outside of the first element (100) to form a axially fixed but rotational free bearing connection. The first element (100) thus can rotate with respect to the release button (4) and since the release button (4) is rotationally fixed to the dose setting element (2), the first element (100) is thus also rotatable with respect to the dose setting element (2).

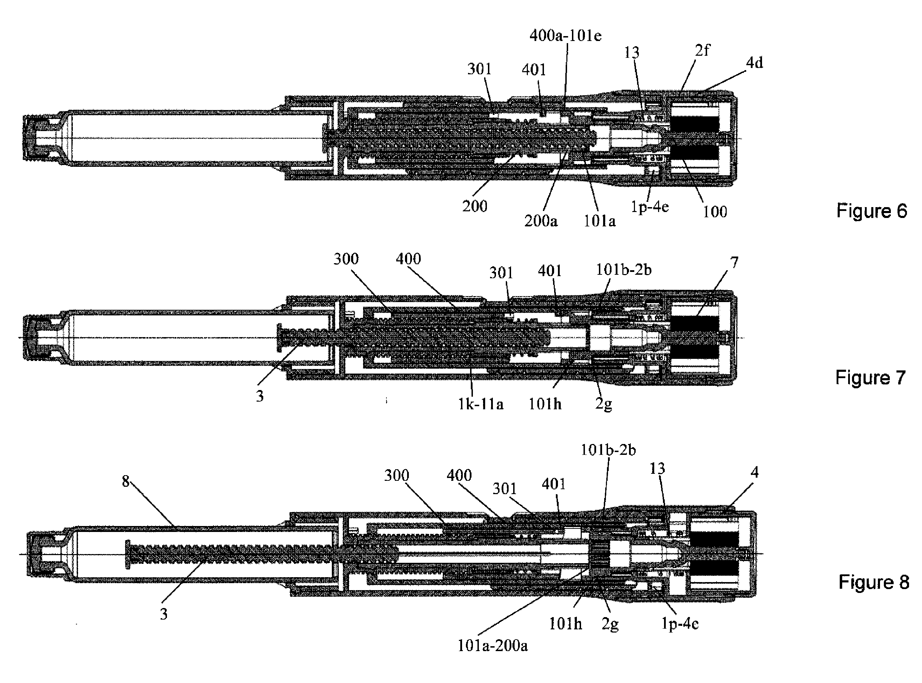

The dose setting element (2) is during the dose setting step when the release button (4) is in the non-actuated position axially coupled to the housing but it can rotate in both directions with respect to the housing. The release button (4) is both in the actuated and the non-actuated position rotationally coupled to the dose setting element but is allowed to axially move repeatedly between the non-actuated and the actuated position via the fifth linear guidance (4d-2f). During actuation, the release button (4) is pressed and moves against the force of a resilient spring (13) from the non-actuated into the actuated position (compare Figures 5 and 6). Upon release, the release button (4) returns back to the non-actuated position. During the actuation step, a third coupling (1 p-4e) structure is closed that rotationally couples the dose setting element (2) to the housing (Figure 5). A fifth coupling structure or linear guidance (1p) is present on the inside of the proximal rim (1m) of the housing (1) which guidance is oriented along the longitudinal axis of the device. A corresponding sixth coupling structure (4e) exists on the release button (4), for example as axial elements that protrude in the distal direction starting from the circumferential rim (4a). The sixth coupling structure (4e) preferably protrudes though corresponding openings in the proximal rim (2e) of the dose setting element (2) and upon activation of the actuation button, the fifth coupling structure (1p) on the housing can engage the sixth coupling structure (4e) on the actuation button (4) and therewith rotationally lock the housing (1), the dose setting element (2) and the actuation button (4) to each other. The spring force, i.e. torque of the drive spring (7) is, in the actuated position of the actuation button connected through the dose setting element- actuation button assembly to the housing.

The first element (100) comprises a drive sleeve shaft (101d) that protrudes the release button (4) on the proximal side. At the distal end, the first element has a drive sleeve hollow tube (101f) that is permanently connected to the drive sleeve shaft (101d, Figure 2). The drive sleeve shaft and hollow tube are formed as a single part and are aligned parallel to the longitudinal axis (1q). At the distal end of the drive sleeve shaft, the shaft has a contact surface (101c) that is enclosed by a circular protrusion on the inside of the release button (5), and the contact surfaces (5a-101c) ensure that axial forces are transferred from the release button (5) to the drive sleeve shaft (101d) and finally to the first element (100) (Figure 2). A drive spring (7) is operationally coupled between the drive sleeve shaft (101d) and the release button (4). The drive spring (7) is either preloaded or is loaded during each dose setting step. In either case, a torque is exerted between the release button (4), and the drive sleeve shaft and therewith also the first element (100).

The drive sleeve tube (101f) of the first element (100) has on its distal side a fourth coupling structure (101b). The fourth coupling structure (101b) is preferably shaped as longitudinal grooves or notches that are present on the outside of the drive sleeve tube (101f) and which are oriented along the longitudinal axis (1q). The fourth coupling structure (101b) forms together with the third coupling structure (2b) of the dose setting element (2) the second coupling (101b-2b, Figure 4). In the closed position, the second coupling (101b-2b) prevents relative rotations between the first element (100) and the dose setting element (2). The second coupling can be decoupled by axially moving the first element (100) in the distal direction and thereby decoupling the second coupling (101 b-2b) and allowing for relative rotations between the first element (100) and the dose setting element (2). Since the release button (4) is rotationally coupled to the dose setting element (2) independent from whether the release button is in the actuated or non-actuated position, the second coupling allows for relative rotations between the first element and the release button (4) when the second coupling is decoupled. In the coupled state of the second coupling, e.g. when the release button is in the non-actuated position, the spring forces of drive spring (7) are guided from the release button (4) to the first element (100) and via the second coupling to the dose setting element (2). In the non-actuated postion of the release button (4), the spring force, i.e. torque of the drive spring (7) is led through elements which are in a positive fit, e.g. rationally coupled o each other and consequently the torque is not transferred in a rotation of the first element (100) as is shown in Figures 2 and 3.

The first element (100) is axially coupled to the release button (4,5) via a bearing (4g-101i) which is composed of a circumferential rim (4g) of the release button that engages a notch (101i) of the first element. The engagement of the bearing is designed such that a sloped rim (4g) engages the notch during the assembly to ensure a one-way assembly. Pressing the release button (4) against a reset spring (13) moves the release button and the first element in the distal direction from the non-actuated into the actuated position. Upon release of the button (4), the reset spring (13) returns the button and the first element (100) back into the non-actuated position. A rim shaped radial extension (101 h) of the first element abuts the distal end (2g) of the dose setting element (2) once the first element (100) returns into the non-actuated state (compare Figures 7 and 8).

At the distal end of the first element (100) there is a linear guide (101e) that ensures that rotations during the dose setting process and also during the dose delivery process are transferred from the first element (100) to a first stop element (400). In the first embodiment, the linear guide of the first element or drive sleeve (100) is shaped as projections (101e) that are oriented parallel to the longitudinal axis (Figure 5). The projections (101e) match complementary grooves or linear guides (400a) on the first stop element (400) and the engagement (400a-101e) is called a first linear guidance (Figure 6). The guidance transfers the rotations of the first element (100) to the first stop element (400) but allowing for relative axial movements between the two parts.

A first coupling structure (101a) is part of, and located inside of the distal section of the drive sleeve tube (101f). The first coupling structure (101a) is preferably shaped as longitudinally oriented keys that can match complementary longitudinal keyways or grooves on a second element or coupling sleeve (200). (Figure 3). The longitudinal grooves or keyways on the second element (200) act as a first coupling structure (200a) and are part of a first coupling (101 a-200a). When the first coupling (101a-200a) is in the decoupled state, the first element (100) and second element (200) are rotationally decoupled whereas in the coupled state the first and second element are rotationally coupled. The first coupling (101a-200a) is during dose setting in the decoupled state and pressing the release button 4 from the non-actuated into the actuated position couples the first coupling (101a-200a).

The second element or coupling sleeve (200) is a longitudinal sleeve coaxially enclosing a delivery means or piston rod (3). The second element (200) by itself is coaxially enclosed by the inner housing sleeve (1h) in the distal region of the second element (200). The second element is operationally coupled to the housing (1) or more preferably inner housing sleeve (1 h) such that the second element can rotate in one or both directions with respect to the housing. There is a bearing composed of a bearing rim (200d) on the coupling sleeve and a circumferential recess (11) on the inner housing sleeve (Figure 5) which ensures that the coupling sleeve (200) can rotate but not axially move. The coupling sleeve or second element (200) is keyed to the piston rod (3) by inwardly projecting protrusions that match with longitudinal keyways or guiding notches (3b) on the piston rod (Figure 3). The connection between the piston rod (3) and the coupling sleeve (200) is such that axial movements are allowed but there are no relative rotations. The piston rod (3) has an outer threading (3c) superimposed onto the longitudinal guiding means or notches (3b). The outer threading (3c) of the piston rod (3) matches the internal thread (1a) of the housing such that a rotation of the piston rod (3) in the second direction screws and advances the piston rod in the distal direction through the housing for dose delivery. The piston rod (3) has a distal flange (3a) which abuts a stopper in the cartridge and advancement of the flange reduces the volume in the cartridge (9).

The second element (200) or coupling sleeve has a linear guide (200c) present on the outside of the guide sleeve and which is composed of longitudinally arranged grooves or protrusions. The linear guide (200c) of the coupling sleeve is engaged with complementary structures or grooves (11b) on the inside of a reverse lock sleeve (11) such that there is a rotational stable but axially slideable connection between the reverse lock sleeve (11) and the second element or coupling sleeve (200). The reverse lock sleeve (11) has a cylindrical shape and, preferably, coaxially surrounds the coupling sleeve. On the distal end of the reverse lock sleeve (11), reverse lock ratchet elements (11a) are, preferably pointing in the distal direction. As an alternative, the elements (11a) can also point in the radial direction either inwards or outwards with respect to the central axis of the device (1q). The reverse lock ratchet elements (11a) can, for example be composed of asymmetric saw tooth structures that match complementary saw tooth structures or ratchet elements (1 k) which are, for example, part of the housing or are part of the inner housing sleeve (1 h) (Figure 3). The ratchet system (1k-11a) ensures that the reverse lock sleeve (11) element can only rotate in one direction, preferably the second rotation direction, which is opposite to the dose setting direction. Ratchet system (1k-11a) is biased by a biasing means, preferably by a bias spring (12). The bias spring (12) is positioned between a circumferential rim (200b) of the second element and the proximal end of the reverse lock sleeve (11) such that the reverse lock sleeve and ratchet element (11a) are biased towards the ratchet element (1k) of the housing inner sleeve (1 h). The ratchet system thus prevents rotation of the reverse lock sleeve (11) in the first rotation direction and produces audible clicks when the reverse lock sleeve rotates in the second direction, thereby producing the dose delivery click. The reverse lock sleeve (11) is keyed to the second element (200) such that there are no relative rotations allowed. Therefore, the coupling sleeve (200) can also rotate in one direction only, preferably the second direction required for dose delivery. The piston rod (3) is rotationally locked to the coupling sleeve (200) and therefore also the piston rod (3) can rotate in the one direction only that is allowed by the reverse lock ratchet. Opposite rotation of the piston rod in the first rotation direction which would result in a reverse movement of the piston rod is therefore prevented. Moreover, the ratchet system (1k-11a) is designed such that a minimum torque needs to be overcome before a rotation of the reverse lock sleeve (11) in the second direction is allowed. The minimum torque level is required to compensate for parasitic or frictional torques potentially present in the second rotation direction during a dose setting operation and to prevent the reverse lock sleeve (11) from rotation during dose setting in both rotation directions and to allow for one, preferably the second rotation direction during dose delivery.

A second stop element or stop sleeve (300) is coupled to the reverse lock sleeve (11) by a fourth linear guidance (300a-11c) which ensures that rotations of the reverse lock sleeve are transmitted to the second stop element (300) while allowing for relative axial movements. Therefore, the reverse lock sleeve (11) has a longitudinal projection (11c) on the outside that matches a groove (300a) on the inside of the second stop element (300). The second stop element (300) is a sleeve that is threadedly engaged via an internal threading (300b) to a thread element (1d) of the housing or inner housing (Figure 3). The second stop sleeve (300) has, preferably, on its proximal end a second limiter (301) which, once engaged with a counter limiter (401) that is preferably arranged on the first stop element (400), creates a unidirectional stop, and prevents relative rotation between the first and second stop elements (300,400) in one direction and allows for a relative rotation in the opposite direction. The arrangement of the fourth (300a-11c) guidance structure ensures that the second stop element (300) can rotate in one direction only and that is preferably the second direction for dose correction and the opposite rotation direction is prevented by the reverse lock ratchet (11). Rotation of the second stop element (300) results in an axial movement of the second stop element due to the threaded engagement with the thread element (1 d) of the inner sleeve of the housing. Such axial movement due to the rotation in the second direction preferably results in a shift of the second stop element (300) in the proximal direction. During the dose setting step, the second element (200) does not rotate and consequently also the second stop element (300) does not rotate nor axially move. During dose delivery, the second element (200) is coupled to the first element (100) and rotations, preferably in the second direction are transferred to the second element (200) and therewith also to the second stop element (300). As a consequence, the second stop element axially shifts during the dose delivery step whereas it does not axially shift with respect to the housing during the dose setting step. The second limiter (301) follows the axial and rotational movements of the second stop element (300) and thus moves over the same axial and/or angular distance as the second stop element (300).

A first stop element or stop sleeve (400) is shaped as a hollow tube that is oriented parallel to the longitudinal axis (1 q) of the housing, preferably coaxial with the housing. At the distal end, the first stop element (400) has an internal thread segment (400c) that engages the thread element (1d) of the inner housing sleeve (1h) (Figure 5). At the proximal end, the first stop sleeve (400) is axially guided and rotationally coupled to the first element (100) via the first linear guidance (400a-101e). The first linear guidance ensures that both during the dose setting as well as during dose delivery the first stop element (400) follows the rotations in the first or second rotation direction. The rotations of the first stop element (400) result in axial shifts due to its threaded engagement (1d-400c) with the inner housing sleeve (1 h). The first stop element (400) moves from an initial proximal position before setting a dose to a second distal position after a dose has been set by rotating the dose setting element (2) in the first rotation direction (compare for example Figures 2 and 4). The axial or angular distance between the two positions corresponds to a dose currently set. During dose delivery, the first element (100) rotates in the second direction and slaves the first stop element (400) to co-rotate and move back from the second distal position to the same proximal position with respect to the housing as before the dose setting and delivery process, i.e. the initial position. During repeated dose settings and deliveries, the first stop element (400) shifts back and forth between the first proximal position and distal positions whereby each distal position is defined by the specific dosage set. A first limiter (401) is axially and rotationally connected to the first stop element (400), e.g. is an integral part, and thus follows the same axial and rotational movements as the first stop element (400).

The first limiter (401) follows the axial and rotational movements of the first stop element (400) during dose setting and dose dispensing and moves from the first proximal position to a second distal position, or vice versa depending on the pitch of the threaded engagement to the housing. During the dose setting movement, the axial or angular distance between the first limiter (401) and the second limiter (301) is reduced. During dose delivery, the first limiter (401) moves back from a second distal position to the first proximal position (or vice versa, see the above). The second limiter (301) does not move during dose setting but moves and rotates during dose delivery along the same axial distance or rotational angle as the first limiter thereby keeping the relative axial or rotational distance constant. The second limiter (301) can, in the preferred embodiment, move in the proximal direction during dose delivery however, depending on the pitch of the threaded engagement to the housing the second limiter can also move in the distal direction during dose delivery. In this manner, the axial distance between the first and second limiter is reduced after each dose setting and delivery step and once the first limiter and second limiter are engaged, rotation of the first limiter in the first rotation direction is blocked whereas rotation in the second direction is allowed. The first limiter (401) is an integrated part of the first stop element (400) that is rotationally connected to the first element (100) which itself is rotationally coupled to the dose setting element (2) and thus rotation of the dose setting element (2) in the first rotation direction is prevented once the first and second limiters (401,301) are engaged due to a transfer of the torque in the dose setting direction from the dose setting element (2) to the the first element (100) and from the first element (100) to the first stop element (400) and via the abutment of the stop limiters (401,301) to the second stop element (300) and finally to the housing (1) through the reverse lock sleeve or third element (11). By selecting and programming the initial axial or angular distance between the first and second limiters (401,301), or the first and second stop elements (400,300), the accumulated doses that can be dispensed from the device can be selected and programmed, preferably during the assembly process of the device. The first limiter (401) and second limiter (301) are axial and/or radial stop elements that upon abutment engage such that they prevent relative rotations in one direction, which represents the dial up direction of the dose setting element (2). In the current embodiment, the first stop element (400) has a limiter (401) which is a radial stop on the inside of the first stop sleeve which can engage with a radial counter stop at the proximal end of the second stop sleeve (300) (Figure 8).

The dose scale drum (10) has a tubular shape with an external thread (10a) which engages an internal thread (1i) of the housing. Dose scale numbers (10e) are printed on the outside of the dose scale drum along a helical curve such that a rotation of the scale drum (10) shifts the dose dialed into the viewing window (6) of the housing. The dose scale drum (10) is keyed to the first stop element (400) using a second linear guidance (400b-10b). On the inside of the dose scale drum (10) a linear guide (10b) is present which is arranged along the longitudinal axis (1q) of the device. The linear guide (10b) has a complementary guide (400b) on the outside of the first stop element (400). The linear guide system transfers rotations of the first stop element (400) to the scale drum (10) but allows for relative axial movements. Due to the threaded engagement with the housing (1i-10a) a rotation of the scale drum (10) results in a rotation and axial shift of the scale drum with respect to the housing. Preferably, during a dial up rotation of the dose setting element (2), the scale drum (10) moves in the distal direction and during a dial down or dose correction the dose scale drum moves in the proximal direction, compare Figures 2 and 4. The reverse movements of the dose scale drum during dial up or dial down can easily be varied by selecting the the pitch of the threaded engagement (1i-10a) between the dose scale drum and the housing. The axial and rotational movements of the scale drum in the dial up direction are halted once a radial or axial second stop (10d) of the scale drum meets a counterstop element (1j) in the housing or housing part and prevents the setting of an individual dose which, in this example, corresponds to the maximum individual dose that can be set. Correcting a dose and/or limiting a dose delivery below a minimum or zero dose is prevented by another axial or radial stop (10c) present at the proximal end of the dose scale drum that meets a counter stop, preferably on the housing, in the zero dose position. Thus the movements of the dose scale drum (10) are restricted to limit the maximum individual dose that can be dialed whereas the limiter mechanism (300,400) with the first and second limiters prevent the setting of a dose which goes beyond a limit representing accumulated individual doses.

During the dose setting step, the dose setting element (2) is rotated over discrete steps and each step is accompanied by an audible dose setting click. For that purpose, a dose setting clicker is present between the dose setting element (2) and the housing (1). In the first embodiment, flexible arms (2h) are present on the dose setting element that can ratchet over the linear guide structure (1p) present at the proximal end on the inner rim of the housing. The flexible arms are attached to the proximal rim (2c) of the dose setting element (2) and the resilient forces flex the arms radially into a direction that is opposite to the direction pointing to the center of the device, e.g. outwards. At the end of the flexible arm or flexible arms ratchet elements are present that form a bidirectional ratchet system together with the fifth coupling structure (1p). Additionally this ratchet system ratchets the dose setting element (2) in discrete postions or steps.

The setting and delivery of a dose is presented in the following section. The pen in the initial state, e.g. prepared to receive a full cartridge is shown in Figures 2 to 5. The user holds the dose setting element (2) and rotates the element (2) in the first direction with respect to the housing (1) for increasing a dose. The rotation in the first direction is transferred to the first element (100) via the second coupling (101 b-2b) which rotates and slaves the first stop element (400) and dose scale drum (10) through the first guidance (400a-101e) and second guidance (400b-10b) structures, respectively. The dose scale drum (10) rotates and axially advances in the distal direction due to its threaded engagement (1i-10a) with the housing and the user can read the dosage set in the viewing window (6). For correcting a dose the user rotates the dose setting element (2) in the second direction which is opposite to the first direction and rotation and proximal movement of the scale drum (10) displays the corrected dose in the viewing window (6). The dose setting and dose correction can be done in discrete steps and each unit of increase or decrease is notified to the user by dose setting clicks generated by the dose setting ratchet. During the dose setting, the release button (4) is in the non-actuated position and consequently the second coupling (101 b-2b) is closed to guide the spring force of dose spring (7) in a closed system of first element (100) - release button (4) - dose setting element (2) back to the first element. During the dose setting process the spring (7) is loaded or already preloaded and the spring forces cannot be released onto the drive mechanism.

The first element (100) rotates during the setting of the dose in one of the two rotation directions and slaves the first stop element (400) such that rotation of the first stop element (400) moves the first stop element (400) from a first proximal position as shown in Figure 2 to a second distal position as shown in Figure 4. The axial distance or rotational angle travelled by the first stop element (400) corresponds to the dose currently set. The first limiter (401) rotates together with the first stop element and travels along the same rotational angle on a thread of the housing or axial distance in the distal direction.

After setting the dose, the user pushes the release button (4,5) and the release button moves distally from the non-actuated position (Figure 2) to the actuated position (Figure 6). The release button (4,5) is pressed against the force of a resilient spring (13) and the axial forces are transferred to the first element (100) via the contact surface (101c) at the proximal end of the first element (100) and/or the bearing (4g-101 i). Upon distal movement, the first coupling (101a-200a) between the first element (100) and second element (200) is moved from the decoupled into the coupled position. Subsequently, the third coupling (1 p-4e) is closed and the assembly of dose setting element (2) and release button (4) is rotationally coupled to the housing (1). Upon further movement of the release button (4) into the actuated position, the second coupling (101 b-2b) between the first element (100) and the dose setting element (2) is opened and the spring force, i.e. torque of the drive spring (7) is now effective between the housing and the first element (100) which starts rotating in the second direction through bearing (4g-101i). The first and second element (100,200) both rotate in the second rotation direction and slave the piston rod (3) which rotates and screws through the threading (1 a) of the housing to move in the distal direction for dose delivery. The dose scale drum (10) rotates in the second direction and returns from the distal dose setting position back to the proximal position via the first stop element (400) and the first and second linear guidance structures. A countdown of the dosage set can be viewed in the viewing window. The dose scale drum (10) halts and stops rotating once it returns to the zero dose position and the stop of the dose scale drum (10c) abuts a counter stop of the housing. Due to the first and second linear guidance structures, the first stop element (400) and first element (100) stop rotating.

The first stop element (400) rotates in the second direction during dose delivery and returns from a second distal position to the initial first proximal position. During dose delivery, the second stop element (300) rotates in the second direction and moves in the proximal direction whereby the axial and/or angular distance between the first limiter (401) and second limiter (301) remains constant. During dose delivery, the rotation of the coupling sleeve (300) is followed by the reverse lock sleeve (11) which ratchets over the proximal end of the inner housing sleeve (1 h) to produce the delivery clicks using the ratchet (1k-11a). Once the dose has been delivered, the user removes his thumb from the release button (4) which returns from the actuated into the non-actuated position due to the spring (13) between the dose setting element (2) and the release button (4). The first element (100) which is axially coupled to the release button inner sleeve (4f) also returns to the proximal position defined by the abutment of rims (101 h) and (2g) and the first coupling (101a-200a) between the first and second element is returned from the coupled into the decoupled position. The second coupling (101b-2b) between the first element (100) and the dose setting element (2) is coupled and the third coupling (1p-4e) is decoupled. The dose has been injected and the piston rod has advanced in the cartridge as shown in Figure 7.

Repeated setting and delivery of doses results in a pendulum movement relative to the housing of the first stop element (400) between the first proximal position and a second distal position and thereby reducing the axial or angular distance between the first and second limiters during the dose setting process. Once the dose limiter is at the end of the programmed delivery regime, the first and second limiters abut during the last dose setting step and prevent the setting of a dose which goes beyond a predetermined amount of medication. The dose setting element (2) cannot be rotated in the first rotation direction but the set dose can still be dispensed. The final position where the first and second limiters of the first and second stop element abut is presented in Figure 8.

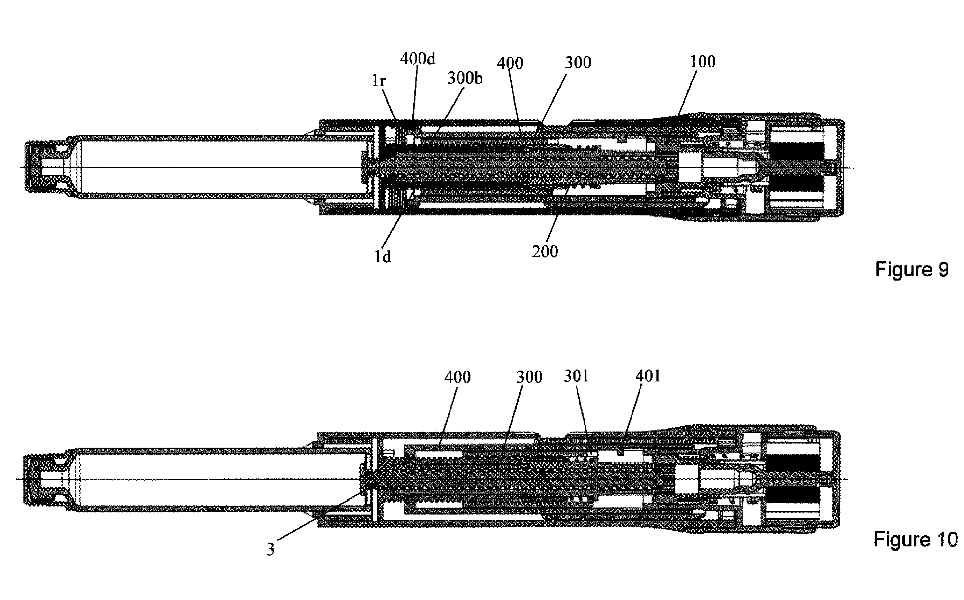

In Figure 9, a second embodiment of the present invention is shown. In principle the dose setting and delivery functions identical to the first embodiment presented above. The first stop element (400) rotates together with the first element (100) during a dose setting step in a first direction whereas the second stop element (300) does not rotate. During dose delivery both the first and the second stop elements rotate in the second direction. The second stop element (300) runs on the thread (1d) of the inner housing sleeve (1 h) via internal thread segment 300b. The first stop element is threadedly engaged, for example, at the distal end, by an external thread segment (400d) with an internal thread (1r) of the outer housing sleeve (1g). The first and the second stop elements (300, 400) thus run each on separate threads, which each can have their own pitches. The fact that the first and second stop elements do not run on the same thread can have advantages for selecting the axial and/or angular distances the parts travel during each dose setting (first stop element) and dose delivery (first and second stop element). In this configuration the axial and/or angular distance between the first and second limiters does not necessarily remain constant during dose delivery.

A third embodiment of the present invention is shown in Figure 10. The parts are identical to the first embodiment shown in Figures 1 to 8 except that the starting position of the second stop element (300) for a new and full pen is chosen to be proximal compared to the starting position in the first embodiment (compare Figures 2 and 10). During repeated dose setting the limiting mechanism works identical to the first embodiment but the different starting position of the second stop element ensures that the setting of a dose is prevented which goes beyond a dispensable amount of medication. The dose limiting mechanism prevents the advancement of the piston rod to the distal end of the cartridge but stops already at an intermediate position. A residual amount of medication will remain in the cartridge when the first and second limiters (301, 401) are engaged. Thus by selecting the initial starting position of the second stop element, the volume that can be dispensed from the nominal volume can be programmed, i.e. restricted to a predetermined value or volume. During the assembly of the dose setting and limiting mechanism, the relative position of the first and second stop elements are selected such that the angular and /or axial distance represents the desired amount of dispensable volume. Thus a method of assembly is presented for a dose setting and limiting mechanism whereby the stop elements are positioned such that a predetermined amount of medication can be dispensed from the device. The predetermined amount of medication may be the nominal volume but can also be a dispensable volume which is below the nominal volume contained in the cartridge. A cartridge holder without a viewing window (not shown here) could be advantageous to prevent users from trying to attempt to set additional doses from the device if they are aware of the presence of the residual volume.

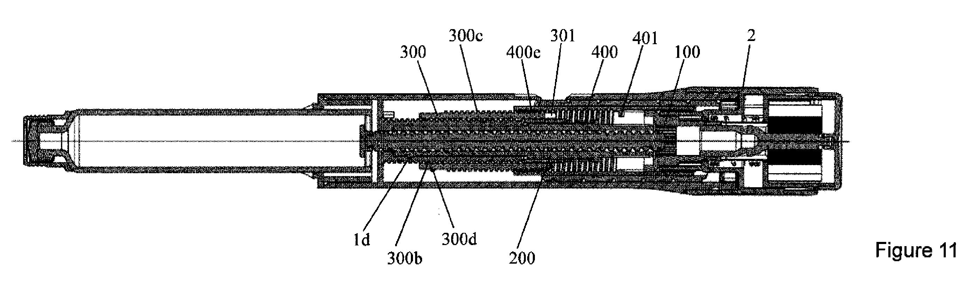

A fourth embodiment of the present invention is presented in Figure 11. The drive and dosing mechanism is, in principle, identical to the first embodiment and the parts can be identified accordingly. The limiting mechanism is based on the first and second stop elements whereby the first element rotates during dose setting and moves during dose setting and the second stop element only rotates during the dose delivery step. The first stop element (400) is guided by a linear, i.e. axial guidance to transmit the rotations of the dose setting element (2) to the first stop element. The first stop element is threadedly engaged to the second stop element (300) which does not rotate during dose setting. As a consequence, the first stop element (400) rotates and translates due to the threaded engagement with the second stop element during setting of a dose. The second stop element (300) has a thread element (300c) on the outside starting at the proximal end and which is complementary to an inside thread element (400e) of the first stop element (400). The first limiter (401) moves along the same axial and/or rotational distance with respect to the second limiter (301), e.g. the distance between the two limiters is reduced during dose setting. During dose delivery, the first and second stop elements (300,400) are rotationally coupled and both rotate in the second rotation direction. The second stop element (300) is threadedly engaged with its internal thread segment (300b) to the thread element (1d) of the inner housing sleeve (1 h) and rotation results in axial movement with respect to the housing (1). The first stop element (400) rotates along the same angle as the second element (200) and there is no relative rotation between the two stop elements (300,400) and both move along the same axial distance with respect to the housing during dose delivery provided that the pitches of the threaded engagements (1d-300b) and (300c-400e) are identical. Alternatively, the pitches of the threaded engagements (1d-300b) and (300c-400e) are not identical and in that case the first and second stop elements (300,400) move each over a different axial distance which is proportional to the angle of rotation needed for dose delivery. After repeated dose settings and deliveries, the first limiter (401) and the second limiter (301) engage or abut and prevent the setting of a dose beyond a predetermined amount. Another option for limiting the relative rotational movements between the first and second limiter is to use the thread element (300c) in combination with thread element 400e. The thread element 300c comprises a thread groove with, for example, a dead end located at the distal end of the second stop element 300. The thread element 400e comprises a thread flank with an end face that, once the end face abuts the dead end of the thread groove prevents further relative rotation in one direction and therewith limiting the setting of a dose beyond a predetermined value. As the first stop element (400) moves in the distal direction with respect to the second stop element (300), the dead end of the thread element (300c) can also provide the stop for limiting the setting of the last dose and prevent that the dose setting element (2) can be rotated beyond a limit. The end of the thread element (300c) together with the complementary internal thread element of the first stop element (400e) thus prevents the setting of a dose beyond a predetermined amount.

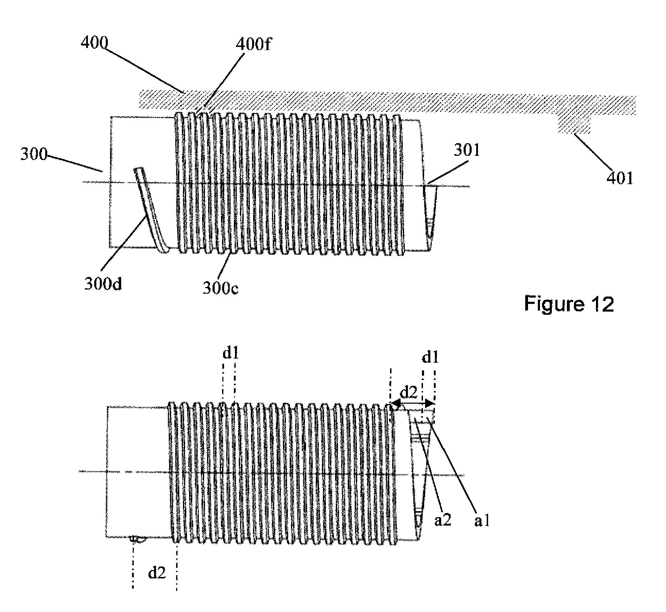

A fifth embodiment of the present invention is shown in Figure 12. The basic mechanism for setting and delivering a dose is identical with the first embodiment except that the first and second stop elements each run on their own thread elements. The second stop element (300) is threadedly engaged with the housing as is shown in the fourth embodiment through the engagement (300b-1d). The first stop element (400) is threadedly engaged with the external thread (300c) of the second stop element (300). The internal thread (400f) is shaped as one or two protrusions or as a short thread segment on the inside of the first stop element (400) which is guided in the external thread (300c) of the second stop element (300). The internal thread (400f) is preferably designed such that it follows a single thread of the external thread segment (300c) of the second stop element (300). Alternatively, the internal thread (400f) is designed to follow two or more thread flanks in case the external thread segment (300c) is composed of a multiple thread. Upon setting the last dose that can be dispensed from the device, the distance between the first and second limiters (301,401) has reduced and engagement or abutment between the limiters prevents further dose setting or rotation of the dose setting element (2) in the first rotation direction. The axial or angular advancement of the first stop element (400) on the second stop element (300) during each rotation is defined by the pitch of the threaded engagement (300c-400f). During the setting of the last dose the overlap (a1) between the first and second limiters, which act as radial stops, e.g. as radially arranged faces, is also defined by the pitch (d1) of the threaded engagement (300c-400f). This overlap defines the final contact area upon abutment between the first and second limiter and in this embodiment it can be an advantage to increase the overlap area upon radial abutment of the first and second limiter. A higher overlap area reduces the contact stresses upon abutment and reduces the risk of plastic deformation or damage of the two limiters (301,401). In the current embodiment this is achieved by increasing the pitch in the final section (300d) of the external thread of the second stop element. During dial up of the last dose, the internal thread (400f) of the first stop element enters the final section (300d) of the thread (300c) of the second stop element (300) and due to the increased pitch (d2) this results in an increased axial movement (d2) of the first stop element (400). Thus during the last part of the rotation that is allowed before abutment of the two limiters, the expected contact area upon abutment between the two limiters is increased from area (a1) to the area (a1+a2), Figure 12.

**List of reference signs**

| | | | |
|---|---|---|---|
| 1 | Housing | 4b | Release button cylinder |
| 1a | internal threading housing | 4c | Contact surface |
| 1b | Circumferential rim | 4d | Linear guide release button (external) |
| 1d | Thread element on inner housing sleeve | 4e | Sixth coupling structure |
| 1g | Outer housing sleeve | 4f | Inner sleeve |
| 1h | Inner housing sleeve | 4g | Rim shaped protrusion, sloped rim |
| 1i | internal thread segment on outer sleeve | 4i | Sleeve connector |
| 1j | Counter stop element | 4j | Outer sleeve |
| 1k | Ratchet element | | |
| 1l | Circumferential recess | 5 | Release button, activation element |
| 1m | Proximal rim housing | 5a | Contact surface |
| 1n | Distal rim housing | | |
| 1p | Fifth coupling structure | 6 | Viewing window |
| 1q | Longitudinal axis housing | | |
| 1r | Internal thread (second embodiment) | 7 | Drive spring, spring |
| 1s | Connector window | | |
| 1t | Notch | 8 | Cartridge holder |
| | | 8a | Needle connector |
| 2 | Dose setting element | 8b | Rim |
| 2a | Distal Projection / rim | | |
| 2b | Third coupling structure | 9 | Cartridge |
| 2c | Circumferential rim | 9a | Septum |
| 2d | Inner sleeve | | |
| 2e | Proximal projection /rim | 10 | Dose scale drum |
| 2f | Linear guide dose setting element (internal) | 10a | External thread |
| 2g | Distal end | 10b | Linear guide scale drum (internal) |
| 2h | Flexible ratchet arm (dose setting click) | 1 Dc | First dose stop scale drum |
| | | 10d | Second dose stop scale drum |
| 3 | Piston rod, delivery means | 10e | Numerical values |
| 3a | Flange | | |
| 3b | Guiding notch, guiding means | 11 | reverse lock sleeve, third element |
| 3c | Thread piston rod | 11a | reverse lock ratchet, dose delivery clicker |
| | | 11b | First linear guide reverse lock sleeve (internal) |
| 4 | Release button sleeve | | |
| 4a | Circumferential rim | | |
| 11c | Second linear guide reverse lock sleeve (external) | 400 | First stop sleeve, first stop element |
| | | 400a | Linear guide first stop sleeve (internal) |
| 12 | Bias spring | 400b | Linear guide first stop sleeve (external) |
| | | 400c | Internal thread segment first stop sleeve |
| 13 | Reset spring | 400d | External thread (second embodiment) |
| | | 400e | Internal thread (fourth embodiment) |
| 100 | First element, drive sleeve | 400f | Internal thread- protrusion (fifth embodiment) |
| 101a | First coupling structure | | |
| 101b | Fourth coupling structure | | |
| 101c | Drive sleeve contact surface | 401 | First stop, first limiter |
| 101d | Drive sleeve shaft | | |
| 101e | Linear guide drive sleeve | 101a-200a | First coupling |
| 101f | Drive sleeve tube | 101b-2b | Second coupling |
| 101h | Rim | 1p - 4e | Third coupling structure |
| 101i | Circumferential notch | | |
| | | 1k -11a | Reverse lock ratchet, dose delivery ratchet |
| 200 | Second element, coupling sleeve | | |
| 200a | Second coupling structure | 400a -101e | First guidance or guiding connection |
| 200b | Circumferential rim | | |
| 200c | Linear guide coupling sleeve | 400b -10b | Second guidance or guiding connection |
| 200d | Bearing rim | | |
| | | 200c -11b | Third guidance or guiding connection |
| 300 | Second stop sleeve, second stop element | | |
| 300a | Linear guide second stop sleeve (internal) | 300a -11c | Fourth guidance or guiding connection |
| 300b | Internal thread segment second stop sleeve | | |
| | | 4d-2f | Fifth linear guidance or guiding connection |
| 300c | External thread (fourth embodiment) | | |
| 300d | End of external thread | | |
| 301 | Second stop, second limiter | | |

## Claims

1. A dose setting mechanism for an injection device for injecting a dose of medication from a reservoir (9) comprising:
a housing (1) comprising a distal end, a proximal end and a longitudinal axis (1q) whereby the reservoir is located near or at the distal end of the housing (1),
a dose-setting element (2) which is rotatable with respect to the housing (1) in a first direction for setting a dose of medication,
a first element (100) operatively coupled with the dose-setting element (2) and which is rotated with respect to the housing (1) in the first direction during dose setting,
and which rotates in a second rotation direction which is opposite to the first rotation direction during dose delivery,
a second element (200) that does not rotate during dose setting with respect to the housing (1) and which is coupled to the first element (100) during dose delivery for rotation in the second direction,
**characterized by**
a first stop element (400) with a first limiter (401), the first stop element (400) being threadedly engaged with one of at least one thread element (1d), the one of at least one thread element (1 d) is provided on a housing part or on a part that does not rotate relative to the housing during dose setting,
and which is operatively engaged with the first element (100) such that the first stop element (400) rotates in the first direction during dose setting and moves along the longitudinal axis (1q) from a first position to a second position due to the threaded engagement with the one of at least one thread element (1d),
and wherein the first stop element (400) rotates in the second direction during dose delivery and moves from the second position back to the first position,
a second stop element (300) with a second limiter (301), the second stop element (300) being threadedly engaged with one of the at least one thread element (1 d) and which is operatively engaged to follow rotations of the second element (200) and which moves along the longitudinal axis (1q) during dose delivery due to the threaded engagement with the one of the least one thread element (1 d), wherein the setting of a dose which exceeds a predetermined amount of medication present in the reservoir (9) is prevented when the first and second limiters (401,301) are engaged.

2. The dose setting mechanism of claim 1 comprising a third element (11) which is operatively coupled between the second element (200) and the housing (1) such that the second element (200) is rotatable with respect to the housing in the second direction and non-rotatable with respect to the housing in the first direction.

3. The dose setting mechanism of claim 2 whereby the third element (11) comprises a one-way ratchet or a two way clutch.

4. The dose setting mechanism of claim 1 wherein the axial or angular distance between the first limiter (401) of the first stop element (400) and the second limiter (301) of the second stop element (300) reduces during dose setting and remains constant during dose delivery.

5. The dose setting mechanism of claim 1 wherein the first stop element (400) and the second stop element (300) each have thread segments (300b, 400c) that engage with the same of the one of the at least one thread element (1d).

6. The dose setting mechanism of claim 1 wherein the first stop limiter (401) is integrally formed, or provided on the first stop element (400) and designed as an axial or radial stop and/or counterstop, and wherein the second stop limiter (301) is integrally formed, or provided on the second stop element (300) and designed as an axial or radial stop and/or counterstop.

7. The dose setting mechanism of claim 1 wherein the predetermined amount of medication present in the reservoir (9) corresponds to the nominal volume of medication contained in the reservoir, or wherein the predetermined amount of medication present in the reservoir (9) corresponds to a dispensable volume from the nominal volume, which is below the nominal volume of medication present in the reservoir (9).

8. The dose setting mechanism of claim 1 wherein the first and the second stop elements (400, 300) are threadedly engaged with the same thread element of the one of at least one thread element (1 d),or wherein each of the first and the second stop elements (400, 300) is threadedly engaged with two separate thread elements (1 d, 1 r) of at least two thread elements provided on a housing part or on a part that does not rotate relative to the housing during dose setting, and wherein each of the two separate thread elements (1d, 1r) has an identical pitch or a different pitch.

9. The dose setting mechanism of claim 8 wherein one of the two thread elements (1 d, 1r) is located on one of the first stop element (400) or second stop element (300).

10. An injection device for injecting a dose of medication having the dose setting mechanism of one of the preceding claims, wherein the second element (200) is operatively engaged with a piston rod (3) such that a rotation of the second element (200) advances the piston rod (3) for injecting a dose of medication.

11. An injection device of claim 10 wherein the second element (200) is splined to the piston rod (3) such that the piston rod (3) and the second element (200) are in a non-rotatable but slidable engagement and wherein the piston rod (3) has an external thread (3c) that is in engagement with an internal threading (1 a) of the housing such that a rotation of the second element (200) rotates and advances the piston rod (3) for delivery of a dose of medication from the reservoir (9).

12. An injection device of claim 10 wherein the piston rod (3) has an external thread (3c) that is threadedly engaged with the second element (200) and wherein the piston rod (3) is non-rotatable and axially slidable engaged with respect to the housing (1) such that a rotation of the second element (200) advances the piston rod (3) for delivery of a dose of medication from the reservoir (9).

13. A method for programming of a dose setting mechanism for an injection device of claims 1 to 9, comprising the steps of selecting a specific initial axial distance or rotational angle between the first limiter (401) of the first stop element (400) and the second limiter (301) of the second element which corresponds to the predetermined amount of medication, positioning the first limiter (401) and the second limiter (301) at the specific initial distance and assembling the dose setting mechanism.
